# EUROPEAN PATENT APPLICATION

(11) **EP 1 657 236 A1**
(43) Date of publication of application: **17.05.2006**
(21) Application number: 04292666.7
(22) Date of filing: 10.11.2004
(51) Int. Cl.: C07C 227/32, C07C 227/18

(54) **Method for preparing diastereoisomers of 4-hydroxy isoleucine**

(71) Applicant: CENTRE NATIONAL DE LA RECHERCHE SCIENTIFIQUE (CNRS), 75794 Paris Cedex 16 (FR); Université Louis Pasteur Strasbourg I, 67000 Strasbourg (FR)
(72) Inventor: Mioskowski, Charles, 67200 Strasbourg (FR); Wagner, Alain, 67000 Strasbourg (FR)
(74) Representative: Peaucelle, Chantal

(57) **Abstract**

The invention relates to a method for preparing diastereoisomers of 4-hydroxy isoleucine of formula A

CO₂H- CH(NH₂)- CH(CH₃)-CH(OH)-CH₃ (A)

which comprises the following steps:
- deprotection of amine of Formula B 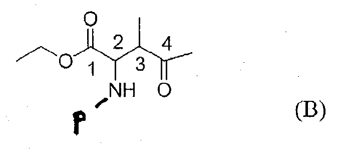
- reduction and subsequent lactonisation of an intermediate amine derivative of formula C 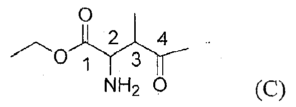 whose substituents at positions 2 and 3 are either R, S or S, S ; or S, R or R, R,
- hydrolysis of the resulting lactone of formula D 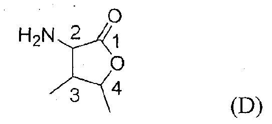
where in substituents at positions 2, 3 and 4 are
- S,R,S or S,R,R when using a protected amine S,R
- S,S,S or S,S,R when using a protected amine S,S
- R,S,R or R,S,S, when using a protected amine R,S
- R,R,R or R,R,S when using a protected amine R,R
under conditions to obtain the desired isomer of 4-hydroxy isoleucine.

Application for preparing the 8 isomeric forms of 4-hydroxy isoleucine.

## Description

The invention relates to a method for preparing diastereoisomers of 4-hydroxy isoleucine. 4-hydroxy isoleucine has the following structure

COOH- C(NH₂)- CH(CH₃) -CH(OH)-CH₃ (A)

Due to the presence of 3 chiral carbons, 4-hydroxy isoleucine will exist under eight isomeric forms, i.e.

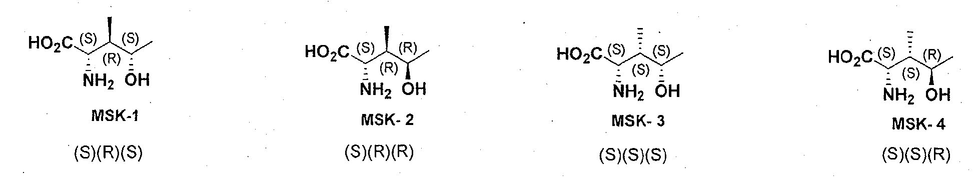

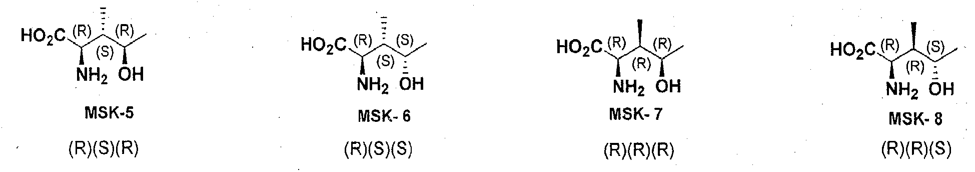

The inventors have developed a method to obtain any one of said isomers, as desired, using the same general reaction scheme, but changing specific condition reactions.

The method of the invention for preparing diastereoisomers, of 4-hydroxy isoleucine, of general formula A

CO₂H- CH(NH₂)- CH(CH₃)-CH(OH)-CH₃ (A)

comprising
- deprotection of amine of Formula B 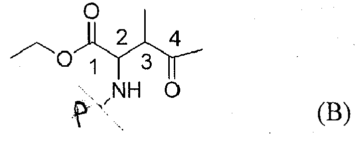
- reduction and subsequent lactonisation of an intermediate amine derivative of formula C 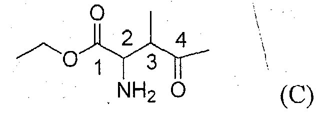 whose substituents at positions 2 and 3 are either R, S or S, S ; or S, R or R, R,
- hydrolysis of the resulting lactone of formula D 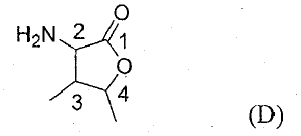 where in substituents at positions 2, 3 and 4 are
   - S,R,S or S,R,R when using a protected amine S,R
   - S,S,S or S,S,R when using a protected amine S,S
   - R,S,R or R,S,S, when using a protected amine R,S
   - R,R,R or R,R,S when using a protected amine R,R
   under conditions to obtain the desired isomer of 4-hydroxy isoleucine.

The invention particularly relates to a method, wherein according to a first embodiment said lactone is obtained in one pot, by oxidative deprotection (step 1) and borohydride reduction (step 2).

In another embodiment, resulting in the obtention of other isomers, said lactone is obtained in two pots by deprotecting the amine with an oxidising agent resulting in an intermediate of formula C which is isolated. 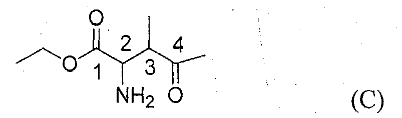 said intermediate derivative C being then submitted to a borohydride reduction to give the desired isomers.
Advantageously, said oxidizing agent is a mild oxidative agent.

Said first embodiment comprises the use of protected amine of formula B, having S, R substituents, respectively at positions 2 and 3, or having S, S substituents, respectively at positions 2 and 3.

Said second embodiment comprises the use of protected amine formula B having R, S substituents, respectively at positions 2 and 3, or R, R substituents, respectively at positions 2 and 3.

Said protected amine R, S or S, R of formula B is more preferably obtained by epimerisation of protected amine S, S or R, R, respectively.

Said epimerisation is advantageously carried out with DBN/t-BuOMe

Preferably, the protected amines (R, S, or S, R; S, S or R, R) of formula B are obtained by condensing a derivative of formula E 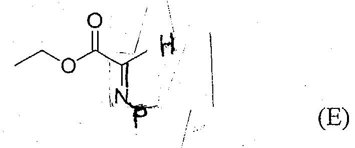 wherein P is an amino-protecting group, such as para-phenylmethoxy group,
with 2-butanone 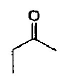 in the presence of proline.

In order to obtain said protected amines S, R, or S, S, L- proline is added to the reaction mixture.

In order to obtain said protected amines R, S, or R, R, D- proline is used instead of L-proline.

In a preferred embodiment of the invention said derivative of formula E is obtained by condensing an amino derivative of formula F

P-NH₂

wherein P is such as above defined,
with ethylglyoxalate of formula G

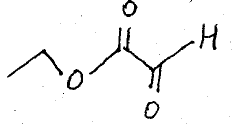

A particularly preferred method for preparing diastereoisomers of 4-hydroxy isoleucine comprises:
- a) condensing an anisidine derivative of formula I 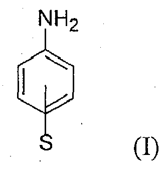 wherein S represents one or several substituents identical or different selected in the group comprising (C1 - C3) alkyl, alkoxy with a (C1 - C3) alkyl group, (C1- C3) aryl or aryloxy, halogen such as F, Cl, Br, I, nitro and nitriles.
   with ethylglyoxalate of formula II 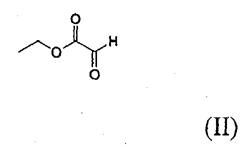 to give imine of formula III 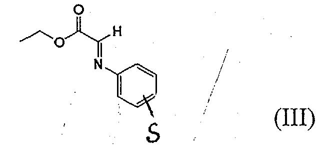
- b) condensing imine of formula III with 2-butanone IV 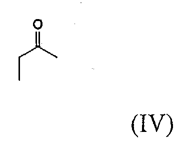 to give a protected amine of formula Va or Vb , 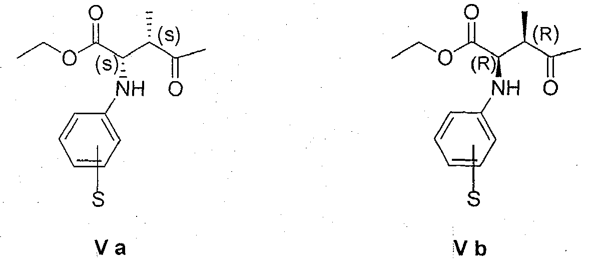 and if desired,
- c) epimerisation of the resulting condensation derivative of formula Va or Vb to give N-protected amine of formula VIa or VIb, respectively, 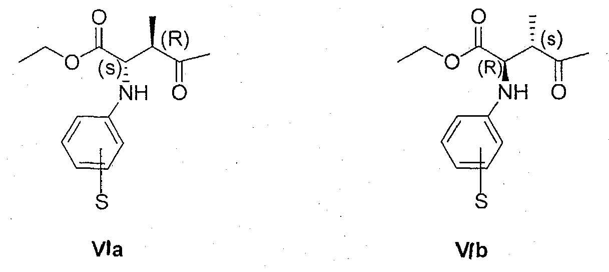
- d) reacting said N-protected amine Va or Vb, or Vla or VIb, under conditions to give -
   - lactone a 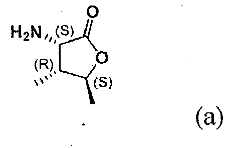 or
   - lactone b 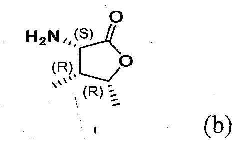 when using N-protected amine VIa
   - lactone c 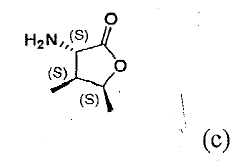 or
   - lactone d 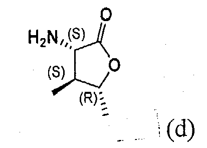 when using N-protected amine Va
   - lactone e 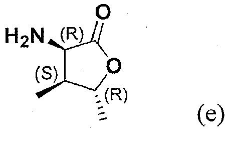 or
   - lactone f 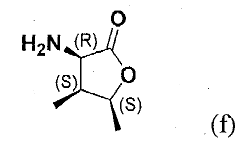 when using N-protected amine VIb
   - lactone g 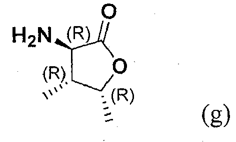 or
   - lactone h 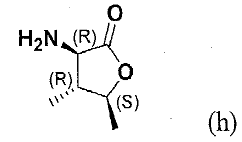 when using N-protected amine Vb
- e) hydrolysing said lactones to obtain the desired stereoisomer..

Step a) is advantageously carried out in an organic solvent such as toluene and all aromatic hydrocarbons, chlorosolvents such as chloroform, dichloromethane, polar protic and aprotic solvents such as DMF, DMSO, acetonitrile, dioxane, ether, THF. In a preferred embodiment, ethylglyoxalate of formula II is added to a solution of the p-anisidine derivative of formula I in said solvent, advantageously containing dehydrating agents, such as Na2SO₄, MgSO₄, K2SO₄, CaCl₂ molecular sieves and azeotropic distillation using aromatic hydrocarbon solvents like toluene, xylene, benzene.

The condensation of imine of formula III with 2-butanone according to step b) is advantageously carried out in presence of an organo catalyst with both antipodes of pyrrolidine 2 carboxylic acid and its derivatives, preferably proline in an organic solvent or mixture of organic solvents, such as DMF, alcohols C1-C7, toluene or aromatic hydrocarbons, chloro solvents such as chloroform, dichloromethane, polar protic and aprotic solvents such as DMSO, acetonitrile, dioxane, ether, THF.

Both antipodes of pyrrolidine -2-carboxylic acid and its derivatives means, for example,

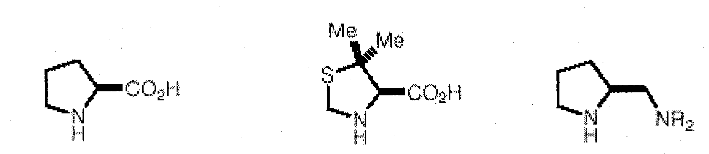

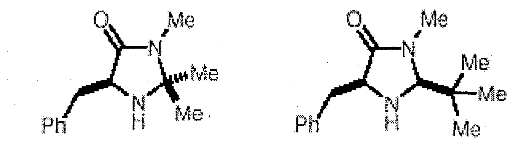

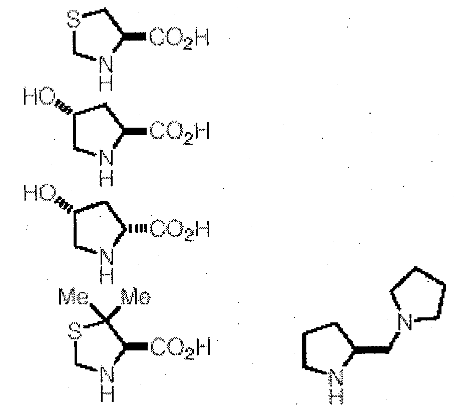

According to step c), epimerisation of the N-protected amine of formula Va or Vb is performed, by adding catalytic amounts of DBN, DBU, DABCO, DEIPA, TEA, Alkyl amines, alkali and alkaline, earth metal alkoxides, aryl amines, and substituted aryl amines, in solvents such as ethers, substituted ethers C1-C7 aromatic hydrocarbons and alcohols C1-C6, to a solution of the N-protected amine in a solvent such as t-BuOMe.

Step d) comprises the obtention of the lactones from the protected amines (VIa or VIb, Va or Vb).

According to a first embodiment, in order to prepare (2S 3R 4S) 4-hydroxy isoleucine isomer (hereinafter MSK-1), step d) is carried out in one pot by reacting protected amine VIa in solution in an organic solvent, with an oxidising reagent at low temperature of-4°C to + 4°C, preferably of about 0°C, neutralizing the reaction mixture at pH 7, adding a borohydride, reducing agent and adding a hydrolysing, to give resulting lactone a reaction mixture.

(2S 3R 4S) 4-hydroxy isoleucine isomer (MSK-1) is recovered after acidification of the reaction mixture, evaporation of the solvents and crystallization.

Said oxidizing agent is advantageously selected in the group comprising Ceric Ammonium Nitrate (CAN), ammonium persulphate, sodium persulphate, potasium persulphate, transition metal oxides and other mild oxidising agents such as lithium perchlorate, sodium hypochlorate.

When the oxidizing agent does not comprise Cerium, said element is further added prior to the reduction.

Said borohydride reducing agent is preferably a metal borohydride of formula M(BH4)n, wherein M is selected in the group comprising sodium, lithium, magnesium, calcium, aluminium, tin, titanium, and said hydrolysing agent is preferably a metal hydroxide such as lithium hydroxide or sodium hydroxide.

According to a second embodiment, in order to prepare (2S, 3R, 4R), 4-hydroxy isoleucine isomer (hereinafter MSK2), step d) is carried out in two pots by reacting protected amine VIa with an oxidising agent such as above defined, neutralizing the reaction mixture at about pH 8, and recovering pure amine of formula VII.

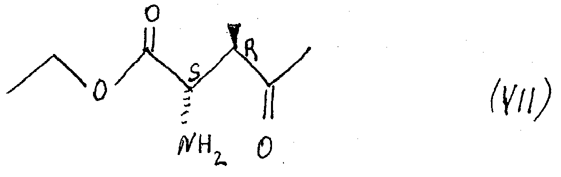

Said amine is then treated with a borohydride reducing agent such as above defined and an hydrolysing agent such as above defined, is added to the resulting lactone b reaction mixture.

(2S 3R 4S) 4-hydroxy isoleucine isomer (MSK-2) is recovered after acidification of the reaction mixture, evaporation of the solvents and crystallization.

According to a third embodiment, in order to prepare (2S, 3S, 4S), 4-hydroxy isoleucine isomer (hereinafter MSK-3), step d) is carried out in one pot, as above defined with respect to step c) except that protected amine Va is used instead of protected amine VIa.
(2S, 3S, 4S) 4-hydroxy isoleucine isomer (MSK-3) is recovered after acidification of the reaction mixture, evaporation of the solvents and crystallization.

According to a fourth embodiment, in order to prepare (2S, 3S, 4R), 4-hydroxy isoleucine isomer (MSK-4), step d) is carried out in two pots, as above defined with respect to the obtention of MSK-2, except that protected amine Va is used instead of protected amine VIa.

(2S, 3S, 4R) 4-hydroxy isoleucine isomer is recovered after acidification of the reaction mixture, evaporation of the solvents and crystallization.

By using the same protocols as above defined with respect to the obtention of MSK-1, MSK-2, MSK-3 and MSK-4, but using protected amines VIb or Vb, respectively, the following isomers are obtained:
(2R, 3S, 4R), 4-hydroxy isoleucine isomer (hereinafter MSK-5),
(2R, 3S, 4S), 4-hydroxy isoleucine isomer (hereinafter MSK-6),
(2R, 3R, 4R), 4-hydroxy isoleucine isomer (hereinafter MSK-7, and
(2R, 3R, 4S), 4-hydroxy isoleucine isomer (hereinafter MSK-8).

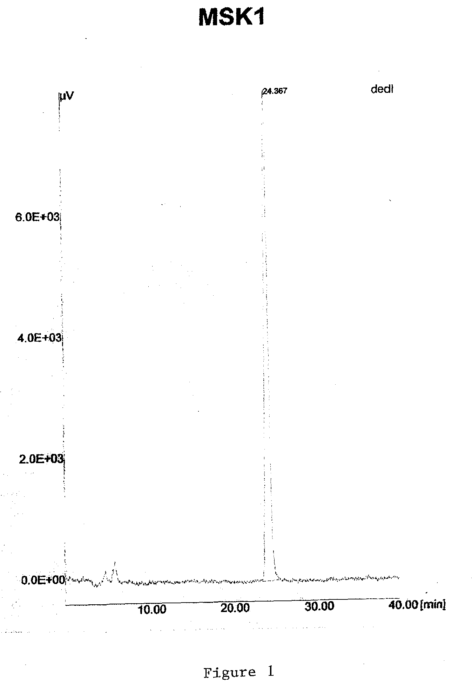

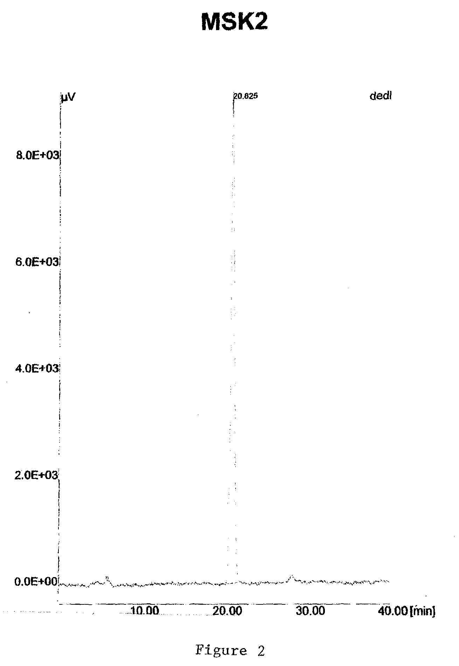

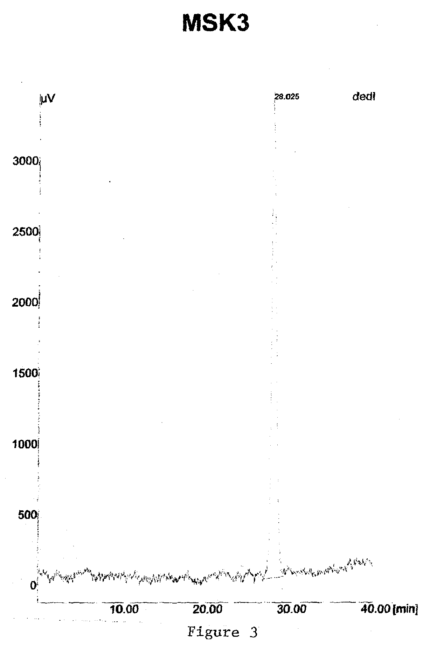

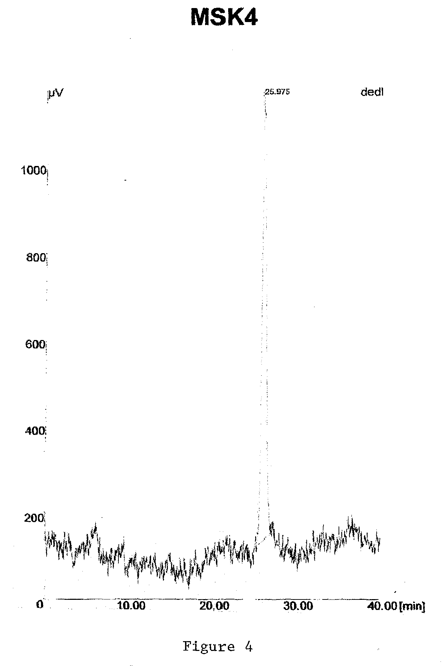

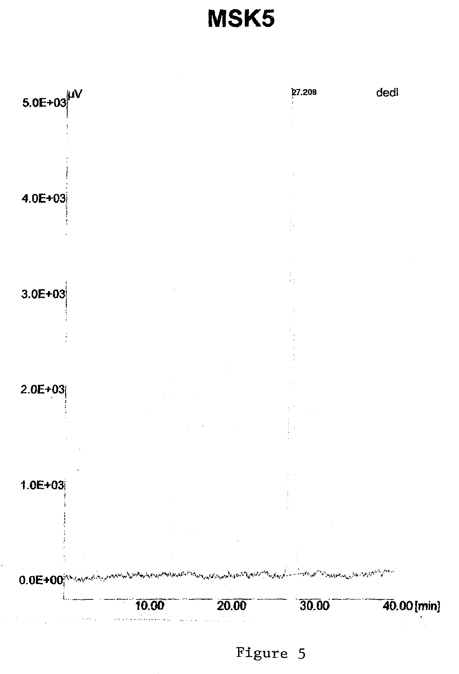

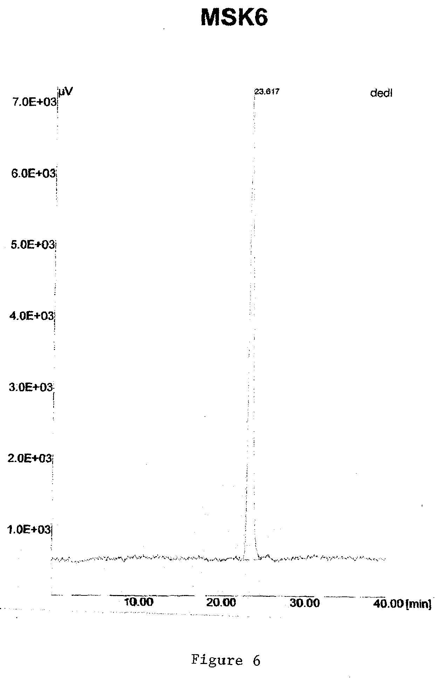

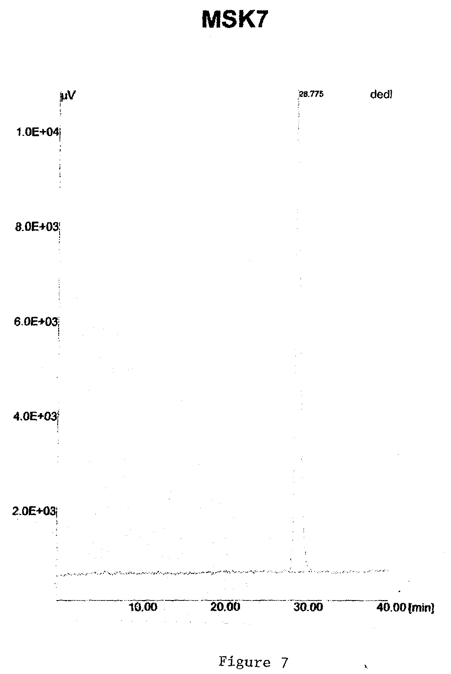

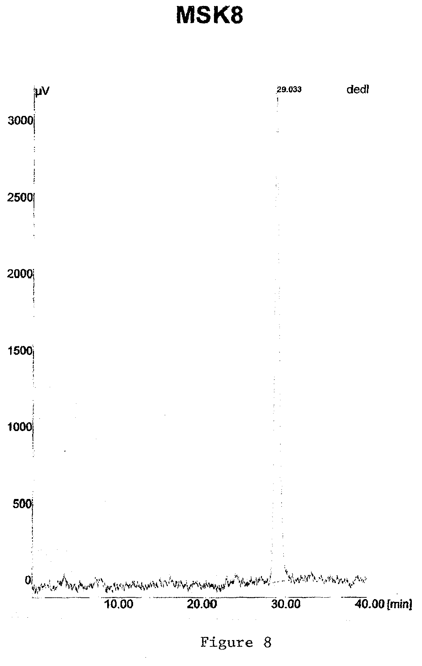

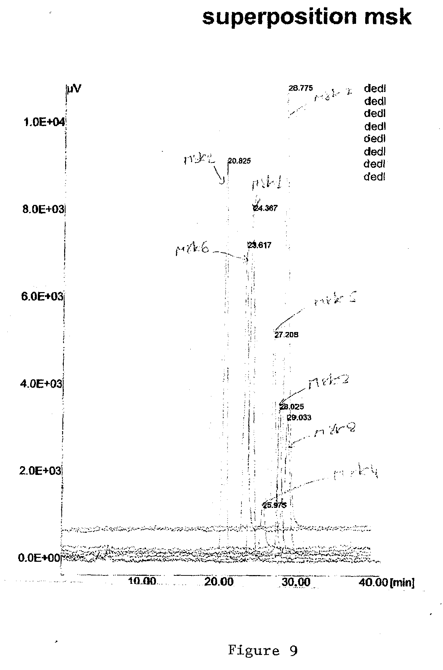

Other characteristics and advantages of the invention will be given hereinafter with reference to figures 1 to 8,which represent, the HPLC of the 8 stereo isomers MSK1-8. The analysis was performed on a Chirobiotic column with 0.5 ml / ml flow, using CH₃CN/H₂O 75/25 at 25° C. DEDL detector was used at 50° C / gain 7/P 3.4 bars Helium, and figure 9 which shows superposition of HPLC curves of MSK1-8.

### Example 1: Preparation of N-pmp amine of formula (5)

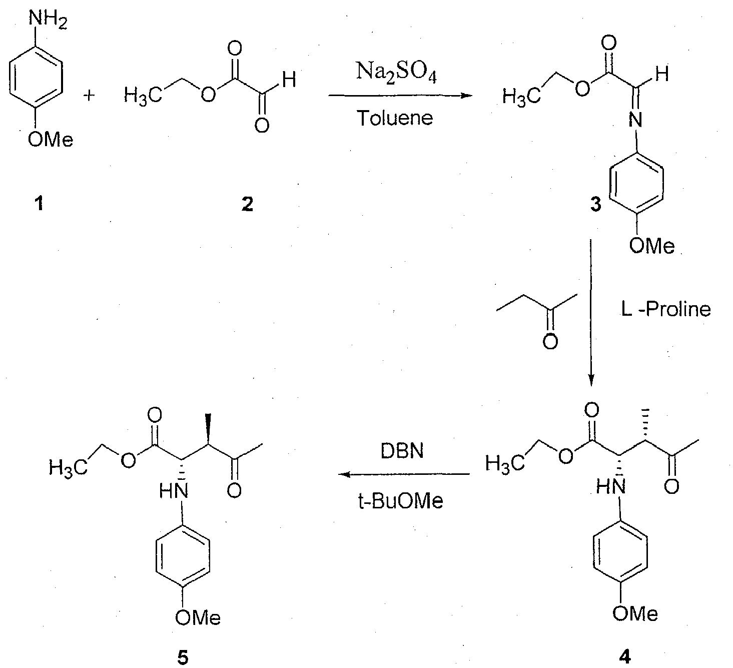

### preparation of imine (3) :

50 g of p-anisidine (1) (406mmol) was dissolved in 400 ml of toluene and 200 g of sodium sulfate (aprox. 2.5 eq) was added and stirred. 82ml of ethyl glyoxalate (2) (50% in toluene 1 eq) was added slowly and proper stirring was maintained. The reaction was completed in 30 min. Sodium sulfate was filtered off using Celite® and toluene was removed under vacuum. 80g (95% yield) of crude product (3) was recovered. The next condensation reaction was carried out on the crude reaction product.

### Synthesis of N-PMP amine ( 5 ):

2-butanone (800ml, 22 eq), dry DMF(600 ml) and L- Proline (15.8 gm, 0.35 eq) were added and stirred at room temperature under nitrogen.

Imine (**3**) obtained in the above reaction was dissolved in 200 ml of dry DMF along with Et₃N (0.40 eq 22.4 ml) and added slowly to the **reaction mixture** (L-Proline + butanone + DMF) under nitrogen and maintained under stirring during about 8h,
L-Proline, butanone and DMF were then successively removed. After complete removal of DMF, the crude mas s (4) (red colored gum) was dissolved in 15 ml of t-BuOMe and 1 ml of DBN (catalytic 0.04 eq) was added and stirred under nitrogen for 2 hrs. The t-BuOMe was allowed to evaporate in overnight at room temperature. After the isomerisation, a solid cake was obtained, dissolved in hot ethanol and crystallized. 48 gms of pure isomerised product (5) were recovered (Total yield: 43%)

### Example 2: Synthesis of (2S, 3R, 4S)- 4-hydroxy Isoleucine MSK-1 :

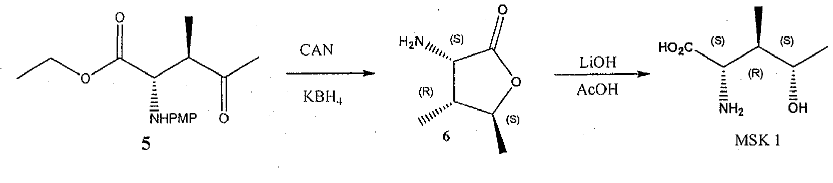

11.6g (40 mmol) of N-pmp protected amine (**5**) was dissolved in 20 ml of acetonitrile. CAN (65.6g, 3eq) in 120ml of water was added with stirring and the reaction temperature inside the flask maintained at 0°C. The initially blue reaction mixture gradually changed to green after complete addition of CAN. The reaction mixture was stirred for 2h30 then extracted with ethylacetate (4x150m1) and the aqueous phase is preserved for the next reduction reaction.

The aqueous phase was neutralised with saturated sodium carbonate and pH adjusted to 7. After neutralisation of the reaction mixture, the reaction flask was cooled to -15°C with proper stirring and maintained for 30 min. Potassium borohydride (3.2g 60mmol 1.5eq) was then added to the reaction mixture.

The reaction was allowed to come to 0°C for about 45min and then basified with 2N Sodium carbonate to pH around 8-9 and extracted with methylene dichloride (5x400ml). The combined organic phases were washed with water, dried over sodium sulfate and evaporated under reduced pressure. 3.73g (62.6% yield) of the resulting lactone mixture (**6**)) was recovered.

The lactone mixture (6) was dissolved in 96 ml of water (0.3 M) and 1.1g (43.3 mmol 1.5eq) of LiOH was added and stirred at room temperature for 2 hrs. The reaction mixture was carefully acidified with AcOH (43.3mmol 2.4 ml). The aqueous phase in the reaction mixture was evaporated in vacuum and ethanol was added and evaporated till all the water was evaporated. The gummy material obtained was crystallised from absolute ethanol.
1.56 g of Isomer (2S 3R 4S) 4-hydroxy Isoleucine was **obtained (purity: 98%) (HPLC).**
The isomer was further purified by preparative HPLC to get white shine powder.

¹**H NMR** (200 MHz, D₂O) δ 3.90 (m, 1H), 3.84 (m, 1H), 1.91(m, 1H), 1.23 (d, *J*=5.6 Hz, 3H) 0.95 (d, *J*=6.6 Hz,3H)
¹³ **C NMR** (75 MHz, D₂O): δ 174.32, 70.46, 57.54, 41.90, 21.30, 12.70.

### Example 3: Synthesis of (2S, 3R, 4R)-4- hydroxy Isoleucine MSK-2 :

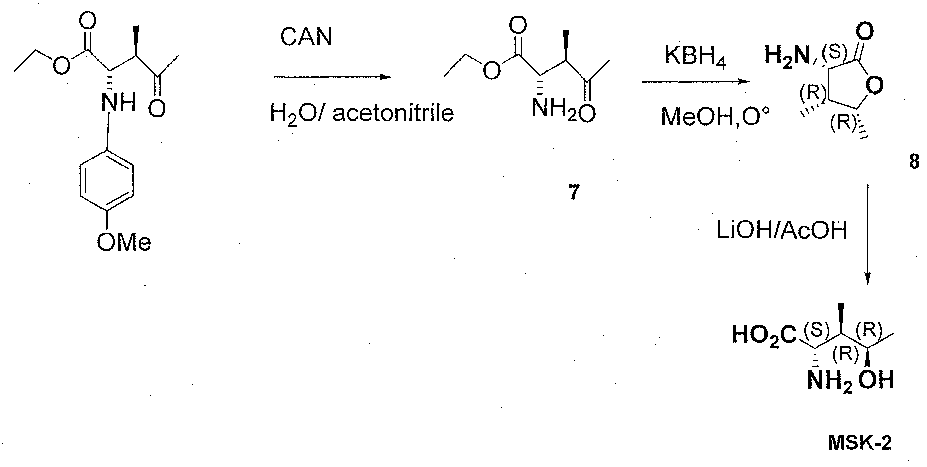

The same procedure as defined in example 2 is applied except that the reaction mixture with CAN is stirred 45 min. instead of 30 min. and the aqueous phase obtained after extraction with ethylacetate is neutralized with saturated sodium bicarbonate solution and made slightly basic (pH is maintained around 8). The aqueous phase is then extracted with 4x 150 ml of dichloromethane. The combined organic extracts were washed with brine, dried over anhydrous sodium sulfate and concentrated under vacuum to yield 5.52g (79.7%) of pure amine (7) as brown coloured oil.

The above-obtained amine (7) was dissolved in 15 ml of methanol and cooled to 0° C and 2.58 g (47.8 mmol) of potassium borohydride solid was added in one time. The reaction mixture is stirred at 0° C for 45 min. and gradually brought to room temperature. Methanol is removed from the reaction mixture in vacuum.

The aqueous phase was extracted with 4x150 ml of dichloromethane. The combined organic phases were washed with brine, dried and evaporated in vacuum to give 2.9g (70.2%) of lactone mixture **(8)** (75:25).

The lactone crude **(8)** was taken up for further reaction without any further purification. The lactone mixture was dissolved in 100 ml of water and 805mg (33.7 mmol) of LiOH added. The reaction is maintained at room temperature for 1 hr and acidified with AcOH (1.91 ml, 33.72 mmol). The water in the reaction mixture was removed under reduced pressure and the crude gummy mass was dissolved in absolute ethanoland again ethyl alcohol was removed .The crude gummy mass thus obtained was dissolved in 90% ethanol and cooled. The separated solid was filtered and washed with ethanol. The grey coloured solid was crystallized with 90% ethanol and yielded 1.4 g (75:25). The sample was further purified with preparative HPLC to get pure white shine material.

### MSK - 2

^{**1**}**H NMR** (300 MHz, D₂O): δ4.05 (m, 1H), 3.80 (d, *J*= 4.2 Hz1H), 2.13 (m, 1H) 1.20 (d, *J*=6.3 Hz, 3H), 1.05 (d, *J*=7.2 Hz,3H)
^{**13**} **C NMR** (75 MHz, D₂O): δ174.49, 69.13, 59.97, 39.12, 20.71, 9.38

### Example 4:Synthesis of (2S,3S,4S)-4- hydroxy Isoleucine: MSK-3.

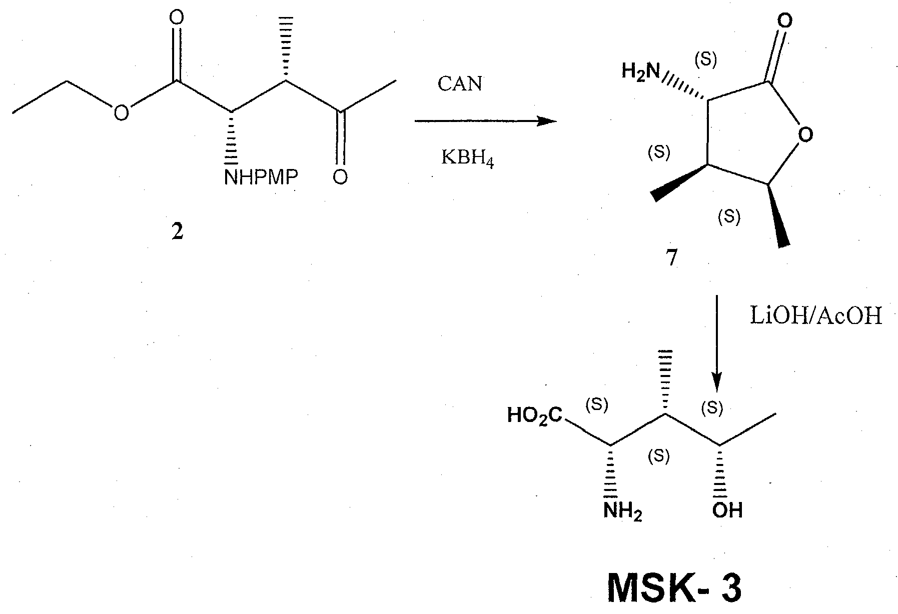

The Mannich condensation product **(2)** was purified by column chromatography to remove regio isomer and p-anisidine using Hex: EtOAc (85:15) as eluent.
5.6g (20mmol) of N-pmp protected amine**( 2)** was *taken in 250 ml round bottomed flask and* was dissolved in 10 ml of acetonitrile. Ammonium nitrate (CAN) (33g, 3eq) in 60 ml of water was added with stirring and maintained the reaction temperature inside the flask at 0°C. The reaction mixture was initially blue in colour and gradually changed to green after complete addition of CAN. The reaction mixture was stirred for 45 mts and checked TLC (hex: EtOAc 7:3) for the presence of starting material. The reaction mixture was extracted with ethylacetate (4x150ml). The aqueous phase was neutralised with saturated sodium carbonate and adjusted pH to 7. After neutralisation of the reaction mixture, the reaction flask was cooled to -15°C with proper stirring. After cooling was maintained for 30 min., 1.6g of potassium borohydride (30 mmol, 1.5eq) was added to the reaction mixture. The reaction was allowed to come to 0°C for about 45min. The reaction mixture was basified further with 2N sodium carbonate to the ph around 8-9 and extracted with methylene dichloride (5x400ml). The combined organic phases were washed with water, dried over sodiumsulfate and evaporated under reduced pressure 1.42 g of lactone mixture.was obtained.

The crude lactone mixture (7) was dissolved in 35 ml of water and 395mg (16.5, mmol 1.5eq) of LiOH was added and stirred at room temperature for two hrs. Tthe reaction mixture is carefully acidified with AcOH (16.5 mmol, 0.9 ml). The aqueous phase in the reaction mixture was evaporated in vacuum and ethanol was added and evaporated till all the water was evaporated. The gummy material obtained was dissolved in 90% ethanol and left overnight. The separated white solid flakes were filtered and washed several times with ethanol to remove lithium acetate impurities. The crude solid was recrystallized with 90% ethanol to give white crystals of (2S, 3S, 4S)-4- hydroxy Isoleucine (MSK- 3), 500 mg.
The sample is further purified by preparative HPLC to get white shine powder.

### MSK-3

^{**1**}**H NMR** (300 MHz, D₂O): δ 4.11 (m, 1H), 3.87 (d, J=2.7 Hz 1H), 2.21 (m, 1H), 1.23 (d, J=6.3 Hz, 3H), 0.92 (d, *J*=7.5 Hz,3H)
¹³ **C NMR** (75 MHz, D₂O): δ174.64, 71.39, 60.39, 38.97, 21.11, 6.19

**Example 5: Synthesis of (2S,3S,4R) -4-hydroxy Isoleucine** 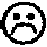 **MSK-4)**

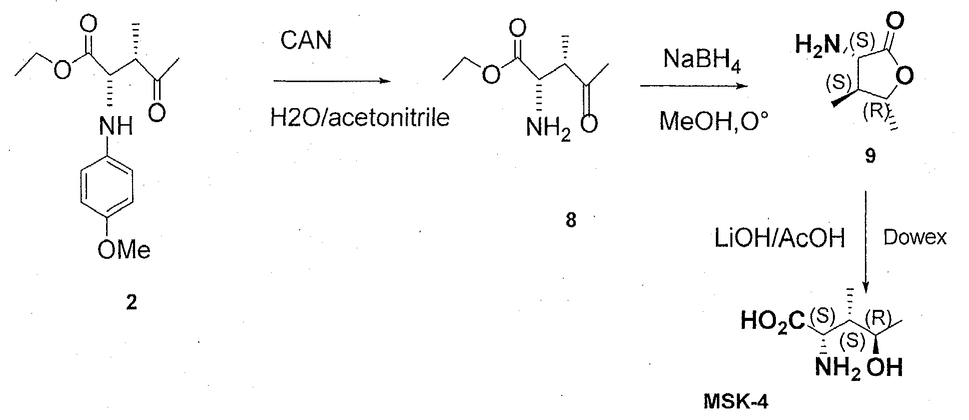

Said reaction is carried out as above mentioned with respect to MSK-2 resulting in pure amine (**10**) as brown coloured oil.

The above-obtained amine (**8**) was dissolved in 15 ml of methanol and cooled to 0° C and 962 mg (1.1 eq, 25. 43 mmol) of sodium borohydride solid was added in one time. The reaction mixture is stirred well at 0°C for 45 min. and gradually brought to room temperature. Methanol was removed from the reaction mixture and diluted with water. The aqueous phase was extracted with 4x 150 ml of dichloromethane. The combined organic phases were washed with brine, dried and evaporated to give 2g of complex mixture lactones.

The lactone crude (15.5 mmol) (**9**) was taken up for further reaction without any further purification The lactone mixture was dissolved in 40 ml of water and to this 556.9 mg (18.6 mmol) of LiOH added .The reaction is maintained at room temperature for 1 hr and is acidified with AcOH (1.31 ml). The water in the reaction mixture was removed under reduced pressure and the crude gummy mass was dissolved in absolute ethanol and said ethanol was further removed .The crude gummy mass thus obtained was dissolved in minimum amount of water and the compound was loaded on a column packed with dowex50wx8 (H+) resine (50 gm). The column was first eluted with water 4x50 ml and then fractions were collected by eluting with 2M ammoniumhydroxide. The ninhydrine positive fractions were combined and evoparated in vaccum to get crude solid free from lithium acetate. The crude solid was dissolved in 90% ethyl alcohol and kept over night. The separated solid (250 mg) was filtered and washed with cold ethyl alcohol and recrystallized from 90% ethanol.

This diastereomer mixture was purified by preparative HPLC to get pure isomer (**MSK-4**) as a white shine powder.

### MSK-4

^{**1**}**H NMR** (300 MHz, D₂O): δ4.02 (d, *J*=3Hz, 1H), 3.81 (m, 1H), 2.12 (m, 1H) 1.28 (d, *J*=6.6 Hz, 3H), 0.97 (d, *J*=7.2 Hz, 3H)
¹³**C NMR** (75MHz, D₂O): δ174.93, 70.18, 56.34, 40.46, 21.24, 12.15

**Examples 6: Synthesis of enantiomers:** The same strategy was employed for the synthesis enantiomers of above described molecules, except that D-Proline was used instead of L-proline in Mannich condensation proline to obtaine the enantiomer.

### MSK-5

^{**1**}**H NMR** (200 MHz, D₂O): δ3.89 (m, 1H), 3.84 (m, 1H),1.90 (m, 1H) 1.23 (d, J=6.4 Hz, 3H) 0.95 (d, J=7 Hz,3H)
^{1**3**}**C NMR** (50 MHz, D₂O): δ174.36, 70.43, 57.51, 41.91, 21.30, 12.6

### MSK-6

^{**1**}**H NMR** (200 MHz, D₂O): δ4.04 (m, 1H), 3.80 (m, 1H), 2.12 (m, 1H), 1.19 (d, *J*=6.2 Hz, ³H) 1.05 (d, *J*=7.2 Hz,3H)
^{**13**} **C NMR** (50 MHz, D₂O): δ 174.55, 69.12, 59.97, 39.12, 20.73, 9.40

### MSK-7

^{**1**}**H NMR** (200 MHz, D₂O): δ 4.10 (m, 1H), 3.87 (d, *J=* 2.6 Hz 1H), 2.23 (m, 1H) 1.23 (d, *J=6.6* Hz, 3H), 0.92 (d, *J*=7.2 Hz,3H)
^{**13**} **CNMR** (50MHz, D₂O): δ 174.64, 71.29, 60.35, 38.96, 21.12, 6.22

### MSK-8

^{**1**}**H NMR** (300 MHz, D₂O): *δ* 4.01 (d, *J*=2.7 Hz, 1H), 3.80 (m, 1H), 2.11 (m, 1H) 1.27 (d, *J*=6.3 Hz, 3H), 0.97 (d, *J*=7.2 Hz, 3H)
^{**13**} **C NMR** (75 MHz, D₂O): δ 174.96, 70.18, 56.35, 40.44, 21.23, 12.10

| **Isomer** | **[α]**^{**21**}_{**D**} **in H**_{**2**}**O** | **m.p** |
|---|---|---|
| **MSK-1** | (+) 30.7 c, 1 | 215-22 ° C (subl.) |
| **MSK-2** | (-) 21.6 c, 0.5 | 202-04 ° C (subl.) |
| **MSK-3** | (+) 28.0 c, 0.25 | 253-55 ° C (dec.) |
| **MSK-4** | (+) 6.0 c, 0.25 | 173-75 ° C |
| **MSK-5** | (-) 31.0 c, 1 | 217-25 ° C (subl.) |
| **MSK-6** | (+) 22.0 c, 0.5 | 200-04 ° C (subl.) |
| **MSK-7** | (-) 30.0 c, 0.25 | 250-54 ° C |
| **MSK-8** | (-) 5.6 c, 0.25 | 173 ° C |

## Claims

1. A method for preparing diastereoisomers of 4-hydroxy isoleucine of formula A
CO₂H- CH(NH₂)- CH(CH₃)-CH(OH)-CH₃ (A)
comprising
- deprotection of amine of Formula B 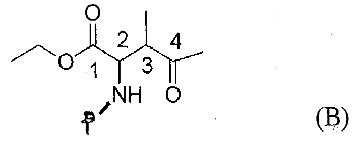
- reduction and subsequent lactonisation of an intermediate amine derivative of formula C 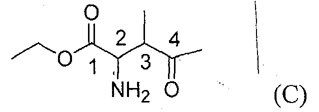 whose substituents at positions 2 and 3 are either R, S or S, S ; or S, R or R, R,
- hydrolysis of the resulting lactone of formula D 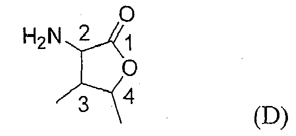 where in substituents at positions 2, 3 and 4 are
- S,R,S or S,R,R when using a protected amine S,R
- S,S,S or S,S,R when using a protected amine S,S
- R,S,R or R,S,S, when using a protected amine R,S
- R,R,R or R,R,S when using a protected amine R,R
under conditions to obtain the desired isomer of 4-hydroxy isoleucine.

2. The method of claim 1, wherein said lactone is obtained in one pot, by oxidative deprotection and borohydride reduction.

3. The method of claim 1, wherein said lactone is obtained in two pots by deprotecting the amine with an oxidising agent resulting in an intermediate of formula C which is isolated 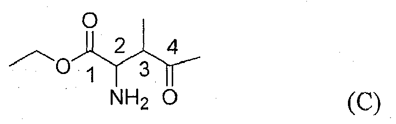 said intermediate derivative C being then submitted to a borohydride reduction reaction to give the desired isomers.

4. The method of anyone of the preceding claims, comprising using said protected amine of formula B, having R, S substituents, respectively at positions 2 and 3.

5. The method of anyone of claims 1 to 3, comprising using said protected amine of formula B, having S, S substituents, respectively at positions 2 and 3.

6. The method of anyone of claims 1 to 3, comprising using said protected amine of formula B, having R, R substituents, respectively at positions 2 and 3.

7. The method of anyone of claims 1 to 3, comprising using said protected amine of formula B, having S,R substituents, respectively at positions 2 and 3.

8. The method of any one of claims 1 to 4, wherein said protected amine R, S or S, R of formula B is obtained by epimerisation of the corresponding protected amine S, S or R, R.

9. The method of claim 8, wherein said epimerisation is carried out with DBN/t-BuOMe.

10. The method of anyone of claims 1 to 9, wherein the protected amines of formula B are obtained by condensing a derivative of formula E 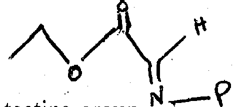 wherein P is an amino-protecting group,
with 2-butanone 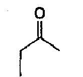 in the presence of proline.

11. The method of claim 10, wherein the proline is L- proline.

12. The method of claim 10, wherein the proline is D- proline.

13. The method of anyone of the preceding claims, wherein the derivative of formula E is obtained by condensing an amino derivative of formula F
P-NH₂
with ethylglyoxalate of formula G

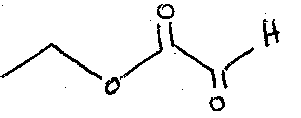

14. The method of anyone of the preceding claims for preparing diastereoisomers of 4-hydroxy isoleucine comprises:
- a) condensing an anisidine derivative of formula I 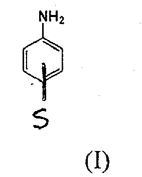 wherein S represents one or several substituents identical or different selected in the group conprising (C1 - C3) alkyl, alkoxy with a (C1 - C3) alkyl group, (C1 - C3) aryl or aryloxy, halogen such as F, Cl, Br, I, nitro and nitriles.
with ethylglyoxalate of formula II 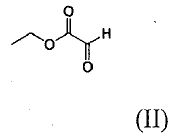 to give imine of formula III 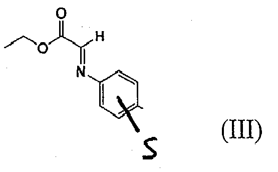
- b) condensing imine of formula III with 2-butanone IV 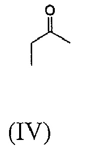 to give a protected amine of formula Va or Vb , 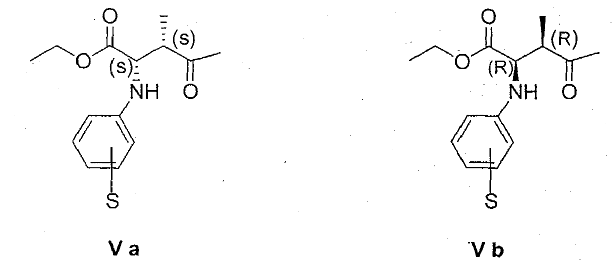 and if desired,
- c) epimerisation of the resulting condensation derivative of formula Va or Vb to give N-protected amine of formula VIa or VIb, respectively, 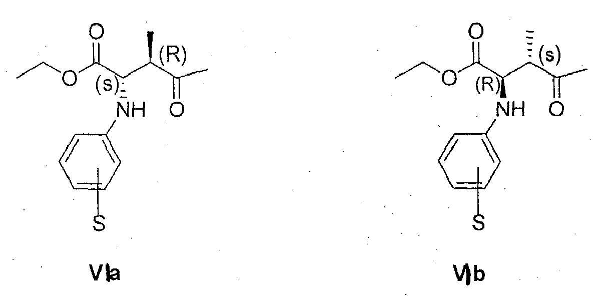
- d) reacting said N-protected amine Va or Vb, or VIa or VIb, under conditions to give
• lactone a 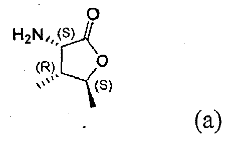 or
• lactone b 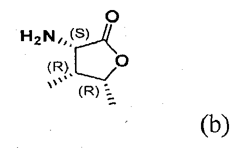 when using N-protected amine VIa
• lactone c 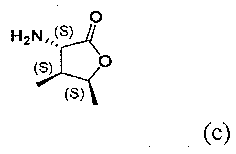 or
• lactone d 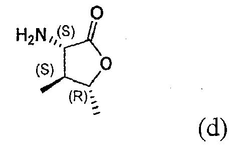 when using N-protected amine Va or
• lactone e 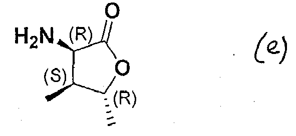 or
• lactone f 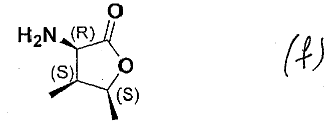 when using N-protected amine VIb
• lactone g 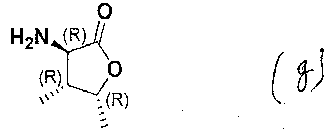 or
• lactone h 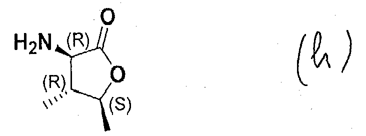 when using N-protected amine VIb
- e) said lactones to obtain the desired stereoisomer.
